# EUROPEAN PATENT SPECIFICATION

(11) **EP 3 796 333 B1**
(45) Date of publication and mention of the grant of the patent: **21.10.2026**
(21) Application number: 20182121.2
(22) Date of filing: 24.06.2020
(51) Int. Cl.: G16H 50/20, G16H 50/50

(54) **UNBIASED ETL SYSTEM FOR TIMED MEDICAL EVENT PREDICTION**
UNVERFÄLSCHTES ETL-SYSTEM FÜR ZEITGESTEUERTE MEDIZINISCHE EREIGNISVORHERSAGE
SYSTÈME ETL SANS BIAIS POUR LA PRÉDICTION D'ÉVÉNEMENTS MÉDICAUX PROGRAMMÉS

(30) Priority: 20.09.2019 US 201962903428 P; 18.06.2020 US 202016905155
(43) Date of publication of application: 24.03.2021
(73) Proprietor: IQVIA Inc., Danbury, CT 06810 (US)
(72) Inventor: LEAVITT, Nadejda, Danbury Connecticut 06810 (US); RIGG, John, Danbury Connecticut 06810 (US); DOYLE, Orla, Danbury Connecticut 06810 (US); NORTH, Benjamin, Danbury Connecticut 06810 (US); WEBBER, Adam, Danbury Connecticut 06810 (US); OZKAN, Emin, Danbury Connecticut 06810 (US); LEE, Suyin, Danbury Connecticut 06810 (US); SALVATELLI, Valentina, Danbury Connecticut 06810 (US); MCLACHLAN, Lachlan, Danbury Connecticut 06810 (US); LONG, Patrick, Danbury Connecticut 06810 (US); CHEHELTANI, Rabe'e, Danbury Connecticut 06810 (US)
(74) Representative: Roos, Peter

(56) References cited:
- US-A1- 2017 083 682
- US-A1- 2018 211 012
- US-A1- 2019 034 590
- US-A1- 2019 172 584

## Description

### BACKGROUND

Extract, transform, load (ETL) typically represents a first step that is utilized in machine learning systems. When applying supervised machine learning classification algorithms to longitudinal healthcare data (e.g. claims data), an important aspect of the ETL process is the creation of labelled patient cohorts - groups of patients who are experiencing similar symptoms which may be monitored over a period of time. Conventional ETL systems typically take a single snapshot of longitudinal healthcare data anchored on a clinical event of interest, such as a diagnosis, and group patients into positive (i.e., diagnosed) and negative (i.e., undiagnosed) cohorts. However, such conventional systems typically use only a single instance of patient medical history which may result in models that may generalize poorly in real-world deployments on new and recent patient data.

US 2019/0172584 A1 relates to individual and cohort pharmacological phenotype prediction platforms and discloses a computer-implemented medical diagnosis using machine learning by means of patients medical histories. A machine learning engine may generate a statistical model based on training data from training patients to predict pharmacological phenotypes.

US 2019/0034590 A1 relates to a system and a method for predicting and summarizing medical events from electronic health records from a multitude of patients. A computer system executes one or more deep learning models trained on the aggregated health records to predict one or more future clinical events.

### SUMMARY

It is an object of the present invention to show a way for achieving a more statistically robust medical prediction model that enables more performant medical event predictions to be made and biases to be substantially reduced or eliminated.

According to the invention, this object is solved by a computing device configured to support an ETL system supporting a timed medical prediction model using machine learning according to claim 1, one or more hardware-based non-transitory computer-readable memory devices storing instructions according to claim 7, and a method implemented on a computing device for generating a dataset for utilization in a machine learning model that is configured to predict one or more medical events of interest for medical patients according to claim 12.

An unbiased ETL (extract, transform, load) system utilizes a rolling series of time-bound cross-sections, termed "rolling cross-sections" (RCS), of patient healthcare data to provide a dataset to a machine learning model for timed medical event prediction. Patients may be labelled as belonging to one of multiple classes (e.g. positive or negative if applied to binary classification) for each cross-section in the series depending on current healthcare status. Rather than using a single snapshot, the unbiased ETL system employs multiple snapshots of patients' medical histories to provide a capability to classify a patient as belonging to one of multiple classes at different points in time, as appropriate. Supervised learning for the machine learning model is thereby enabled over multiple different periods of a patient's medical journey which advantageously supports a more statistically robust medical event prediction model and eliminates several classes of bias. Additionally, the unbiased ETL system enables customization of a prediction window to account for lags in data collection, data processing, and length of use of the medical event predictions, to thereby assure timely and properly utilizable predictions in real-world deployments.

Advantageously, the unbiased ETL system can produce more performant medical event predictions even in health data scenarios with a small sample size for an event of interest, such as diagnoses associated with an ultra-rare disease, as the availability of multiple snapshots of patient data boosts the sample size in the positive cohort, resulting in more information being available for training, testing, and validating the prediction model. Accordingly, computing resources utilized in implementations of machine learning such as processor cycles, memory, power, data transmission bandwidth and storage can be employed with greater efficiency as compared to conventional ETL systems.

These and various other features will be apparent from a reading of the following Detailed Description and a review of the associated drawings.

In an embodiment of the invention, there is provided a computing device configured to support an ETL (extract, transform, load) system supporting a timed medical prediction model using machine learning, comprising:
one or more processors; and
one or more hardware-based non-transitory computer-readable memory devices storing instructions which, when executed by the one or more processors, cause the computing device to:
   collect medical histories for each of a plurality of patients into a dataset;
   create a timeline from the collected medical histories in the dataset in which data for events of interest for the patients are included on the timeline, wherein the events of interest comprise at least one of diagnosis of a disease, progression of a disease, initiation of treatment or therapy, discontinuation of treatment or therapy, occurrence of an adverse event, favorable response to treatment, hospitalization, readmission;
   implement a rolling timebound window into which data is selectively captured as a snapshot of the medical histories of the plurality of patients;
   transform the dataset by rolling the window along the timeline to selectively capture data at different points along the timeline to thereby generate multiple snapshots of the patient medical histories, wherein the multiple snapshots are captured to show a change in therapeutic status from an earlier time to a later time, and
   employ the transformed dataset with the multiple snapshots of patient medical histories in the prediction model to predict one or more medical events of interest,
   wherein the rolling window comprises a lookback window and a prediction window, wherein the prediction window defines the time period over which an event of interest is predicted, wherein the lookback window precedes the prediction window on the timeline and the events of interest occurring within the lookback window are utilized for training of the prediction model, wherein the generated multiple snapshots are utilized to increase sample size.

In an embodiment of the computing device, the events of interest indicate disease progression or change of therapy and the dataset of selectively captured data comprises positive events of interest. In an embodiment of the computing device, the multiple snapshots of patient medical histories enable analysis of a given patient at multiple different points along the timeline. In the computing device according to the invention, the transformation comprises utilizing the generated multiple snapshots to increase sample size. In the computing device according to the invention, it is provided for comprising using the multiple snapshots of patient medical histories in the prediction model to predict one or more medical events of interest. The computing device may be provided for further comprising storing the predictions in a destination system that includes a user interface configured to enable users to interact with the stored predictions. In the computing device according to the invention, the rolling window comprises a lookback window and a prediction window, wherein the lookback window precedes the prediction window on the timeline and the events of interest occurring within the lookback window are utilized for training of the prediction model.

In an embodiment of the invention, there is provided one or more hardware-based non-transitory computer-readable memory devices storing instructions which, when executed by one or more processors disposed in a computing device, cause the computing device to conduct the steps as defined in claim1.

In an embodiment of the one or more hardware-based non-transitory computer-readable memory devices, the prediction window is an element of a cross-section window further comprising a timebound lookback window, the lookback window preceding the prediction window on the timeline, the cross-section window comprising a section of the timeline having a predetermined length, in which clinical events occurring within the lookback window are extracted and utilized for prediction model training. In an embodiment of the one or more memory devices, the cross-section window further comprises an offset window immediately preceding the prediction window on the timeline, the cross-section window comprising a section of the timeline having a predetermined length, in which the offset window is chosen to accommodate time lags in data collection from the data sources. In an embodiment of the one or more memory devices, the final dataset having data captured at multiple different points along the timeline has reduced sampling bias relative to the initial dataset. In this case, the one or more memory devices may be provided in which the sampling bias results from one of seasonality, changes in data coverage, or changes in market conditions. In an embodiment of the one or more memory devices, the executed instructions further cause the computing device to utilize the final dataset to validate the machine learning model or test the machine learning model. In an embodiment of the one or more memory devices, the executed instructions further cause the computing device to use different portions of the final dataset for training to thereby compensate for drift of the machine learning model or bias in the machine learning model.

In an embodiment of the invention, there is provided a method implemented on a computing device for generating a dataset for utilization in a machine learning model that is configured to predict medical events of interest for medical patients, the method comprising the steps as defined in claim 1.

In an embodiment of the method, the different windows have offset start times and end times on the timeline and the events of interest relate to one of disease progression or change of therapy. The method may be provided in which each of the different windows further comprises an offset window between the lookback window and the prediction window on the timeline, in which the offset window provides a predetermined time lag to the prediction window such that the machine learning model is enabled to predict an event of interest by an amount of time equal to the size of the offset window. In an embodiment, the method is provided in which the events of interest are expressed using positive and negative indicators, and in which a positive indicator comprises one of an initiation or escalation of a therapy, a clinical procedure, a diagnosis, or any medical event captured by the available data, or a combination thereof. In an embodiment of the method, it further comprises storing predictions from operations of the machine learning model in a destination system that is configured to interface with one or more computing device users to enable review and analysis of the predictions.

### DESCRIPTION OF THE DRAWINGS

FIG 1 shows an illustrative ETL (extract, transform, load) system that utilizes a machine learning model;
FIG 2 shows an overview of an illustrative approach for implementing an ETL system using a series of timebound rolling snapshots of patient data which may be used in a dataset used by a machine learning model;
FIG 3 shows illustrative examples of rolling cross-sections of patient data that are utilized in an unbiased ETL system for timed medical event prediction which may be used in various implementations of machine learning;
FIG 4 is a graph that illustratively shows how model precision is increased as the prediction window is lengthened;
FIG 5 is a graph that illustratively shows model precision in predicting whether healthcare providers will transition a patient to a specific new therapy within the model's prediction window;
FIGs 6-8 are flowcharts of illustrative methods performed by a computing device or server to implement the present unbiased ETL system for timed medical event prediction;
FIG 9 is a block diagram of an illustrative architecture of a computing system such as a PC (personal computer) or server that may be used at least in part to implement the present unbiased ETL system for timed medical event prediction; and
FIG 10 is a simplified block diagram of an illustrative architecture of a computing device that may be used at least in part to implement the present unbiased ETL system for timed medical event prediction.

Like reference numerals indicate like elements in the drawings. Elements are not drawn to scale unless otherwise indicated.

### DETAILED DESCRIPTION

Conventional ETL (extract, transform, load) systems typically group patients as positive or negative according to their medical histories from a single snapshot. Using a single snapshot approach, patients are indexed on the date of the positive event. Patients may be assigned a fixed lookback period, which can limit the amount of medical history that can be included in the model. Selection of negative cohort is required, and, depending on the selection and matching technique, may lead to introduction of biases into the model.

Instead of using the single snapshot approach, the present unbiased ETL system enables longitudinal transition of patients across one or more classes to provide several advantages over conventional systems (i.e., the system may be configured to support both binary and non-binary classification schemes). By taking samples of the same patients in different snapshots of time, the unbiased ETL system enables training to be performed on different patterns of cross-sectional data without sacrificing the length of longitudinal data that is made available to the system. Training on the different time-bound cross-sections allows for better monitoring and detection of indicators of potential model drift and may substantially reduce or eliminate bias in sampling cohorts that may arise, for example, due to (1) seasonality; (2) changes in data coverage; or (3) changes in market dynamics, such as new market launches or changes in medical protocol.

The unbiased ETL system utilizes multiple snapshots of patients' medical history to enable customized design of a timed window of prediction. The prediction model can be optimized to predict an event of interest for a predetermined number of days in advance. In addition, the prediction is valid for a predetermined number of days. Such customization can provide significant improvements over conventional systems in real-world deployments as deployment strategies can vary by end-user and specific business needs. In addition, the customizable timed window of prediction enables a machine learning system to validate the prediction model based on most recent cross-sections of data which may provide a better indication of how well the model can be generalized to future data.

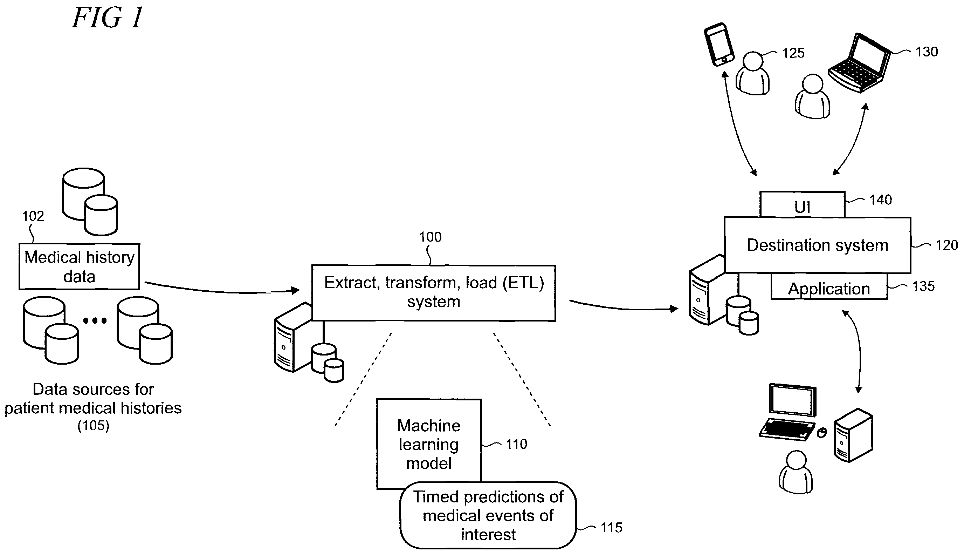

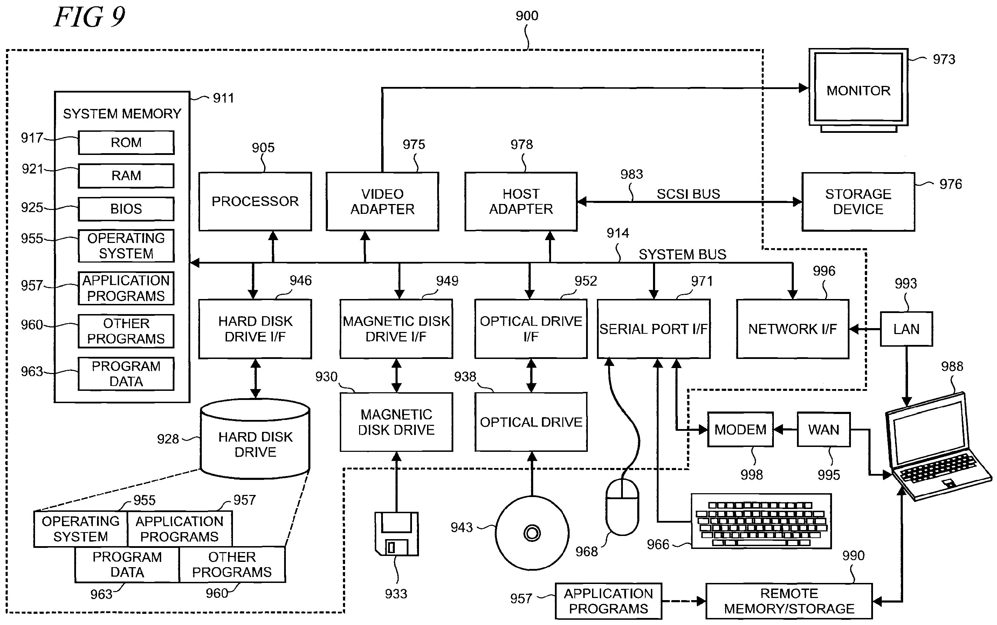

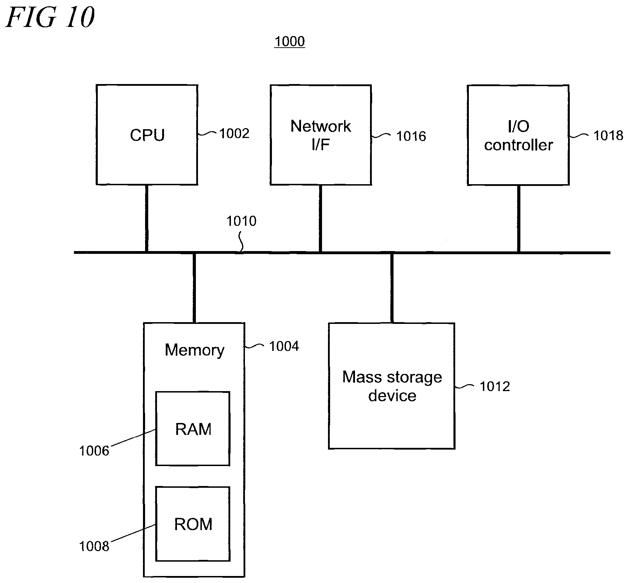

Turning now to the drawings, FIG 1 shows a high level overview of an illustrative ETL (extract, transform, load) system 100 that may be implemented on computing infrastructure, as described below in the description below accompanying FIGs 9 and 10. The ETL system 100 is arranged to receive medical histories of patients from one or more data sources 105. The data sources can be remotely located from the ETL system in some instances, and/or be operatively coupled to the ETL system over one or more communication networks (not shown) including, for example, local area network (LAN) and wide area network (WAN) infrastructure using wired or wireless connections.

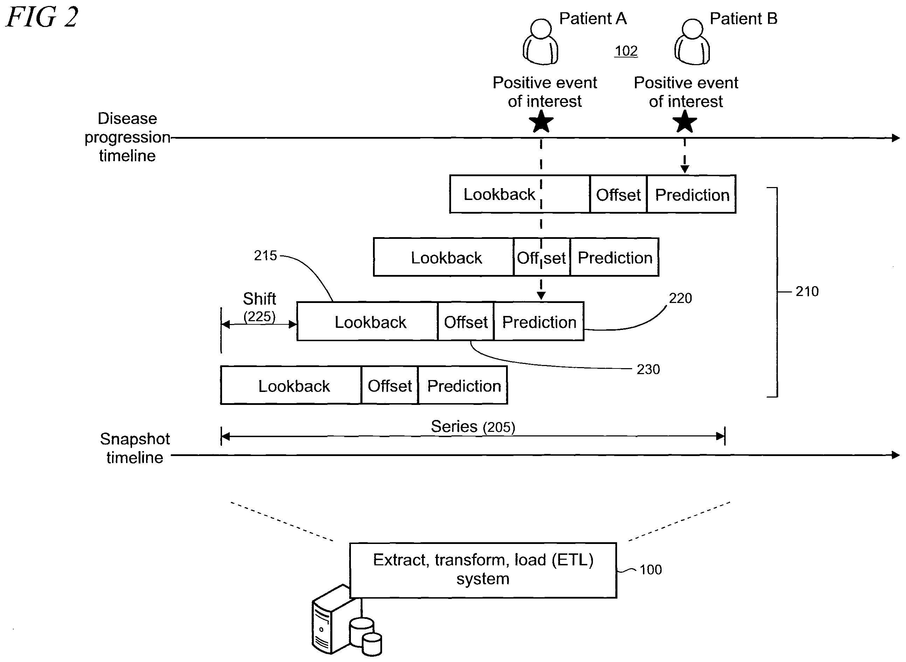

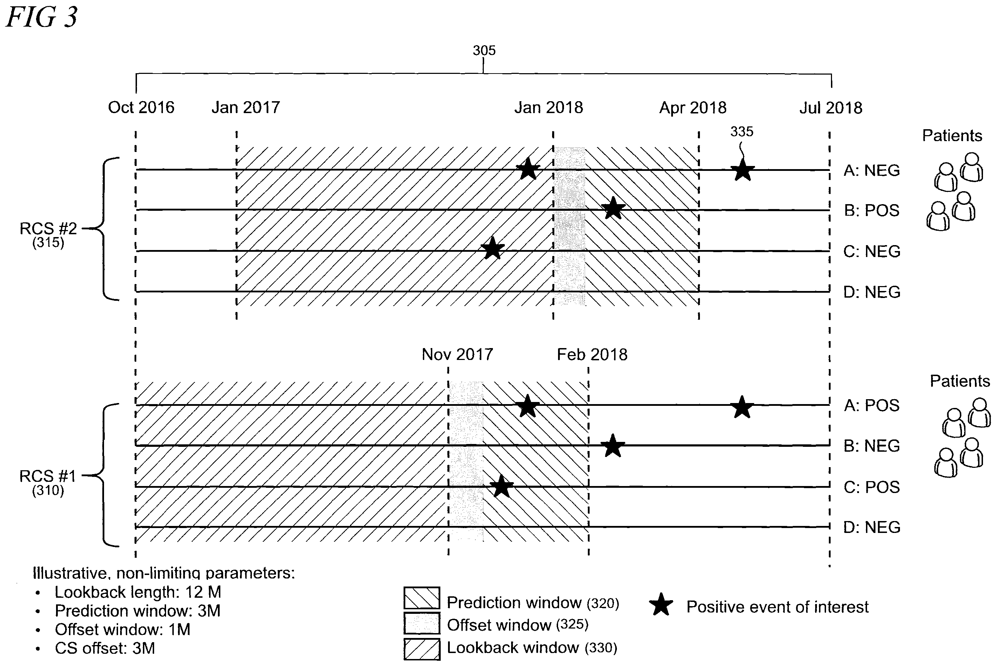

The ETL system 100 supports a machine learning model 110 that is configured to operate on medical history data 102 that is extracted from the data sources 105 and transformed into a dataset using the rolling snapshot approach discussed below in the text accompanying FIGs 2 and 3. The machine learning model provides timed predictions 115 of medical events of interest. As used herein, the term "event(s) of interest" typically relates to medical or clinical contexts and comprise at least one of diagnosis of a disease, progression of a disease, initiation of treatment or therapy, discontinuation of treatment or therapy, occurrence of an adverse event, favorable response to treatment, hospitalization, readmission. The predictions are stored in a destination system 120 that may be locally or remotely instantiated from the ETL system. In some implementations, the ETL and destination systems may be combined in a single system. The ETL and destination systems may be configured to interoperate over a communications network (not shown). Users 125 of computing devices 130 such as personal computers, tablets, and smartphones, can interact with the predictions on the destination system, for example, through an application 135 or user interface (UI) 140 that may support local and/or remote (i.e., cloud-based) usage scenarios.

FIG 2 shows an overview of an illustrative approach for implementing the ETL system 100 using rolling snapshots of patient data which may be used in a dataset used by a machine learning model. The approach facilitates system implementation using a series 205 of timebound rolling snapshots in which patient histories 102 are sub-divided into cross-sections. As shown, each rolling cross-section (RCS) 210 includes a defined lookback window 215, usually on the order of one or two years, from which patient medical history is extracted for model training, as well as a narrow forward-looking prediction window 220 to detect an event of interest, for example, a clinical event defining disease progression, often the initiation or escalation of therapy, that identifies a patient as positive.

The prediction window 220 defines the time period over which an event of interest is predicted, for example, if the window is three months, the model will seek to predict patients that will transition over a three-month period. Thus, the prediction window further provides the time period over which the machine learning model is looking for the events of interest. Successive cross-sections are shifted by a given interval (e.g. monthly increments), as indicated by reference numeral 225 to form a final dataset containing multiple cross-sections of data defined by iterative timeframes of medical history from the initial dataset. An offset window 230 comprises a time period prior to the prediction window that can be incorporated to accommodate lags in data collection. Thus, the machine learning model can predict an event X amount of time in advance, where X is defined by the offset.

A key advantage of this approach is that it captures multiple snapshots of the patient journey in which patients are labelled according to their current therapeutic status within the specific snapshot of time, such as drug initiation versus no initiation. According to this definition, a patient may be considered by the machine learning model as a negative patient during an earlier snapshot of data, and subsequently be considered a positive patient in a later snapshot once the patient has exhibited the event of interest. This enables the machine learning model to learn from more varied and comprehensive representations of patient history regarding events of interest. It also helps overcome challenges arising from small sample sizes, which is often the case for rare events (e.g., a rare disease or adverse event, or the like) and/or niche products, products that have recently launched, or products with narrowly defined market segments, since the number of patient instances used for model training scales in relative proportion to the number of cross-sections. Thus, patients are used more than once for model training, amplifying the signal obtained from each individual patient.

There are several benefits of this multi-snapshot approach to patient data extraction and transformation related to customization that more optimally suits a given clinical scenario or commercial application. For example, the prediction window can be designed such that patient predictions are valid for a given period, for example, a 3-month window. In addition, the offset period prior to the prediction window can be incorporated to accommodate lags in data collection, data processing, or the mobilization of clinical or commercial resources. For example, a 1-month time period prior to operationalizing machine learning model predictions may be used for such offset. Moreover, the opportunity to train on multiple cross-sections allows for better monitoring of indicators of model drift - reduced performance due to market or other changes in the data - allowing for mitigation of temporal biases in patient sampling due to seasonality, fluctuations in data coverage over time, or shifts in market dynamics such as, for example, changing treatment guidelines or regulations.

The multi-snapshot approach to patient data extraction and transformation also enables model validation strategies that evaluate model performance exclusively on "future" data. Specifically, a model can be trained on the bulk of historical medical history snapshots and validated only on the most recent snapshots to produce representative indicators of model performance after real-world commercial deployment. Within this framework, the model is evaluated on data it has not seen from a future time period, as it is trained exclusively on earlier snapshots of data.

FIG 3 shows an overview of one illustrative embodiment of an unbiased ETL system 100 (FIG 1) for timed medical event prediction using a pipeline. An ETL pipeline 305 in this example comprises a timeline that illustratively extends from October 2016 to July 2018 and defines a study period. Two representative RCS (rolling cross-section) instances - RCS #1 and RCS #2 (respectively indicated by reference numerals 310 and 315) - show data for four patients A, B, C, and D on the horizontal lines in rows. The vertical dashed lines on the sections represent various time stamps.

Each RCS includes a prediction window 320, an offset window 325, and a lookback window 330. As noted above, the prediction window includes a time period in which medical events of interest are examined for inclusion in the prediction model supported by the unbiased ETL system.

The offset window 325 provides a portion of a patient's medical history that is not introduced in the prediction model for that RCS to account for data that is not captured in an actual deployment due to data lags and processing times. For example, data lag can occur due to constraints from a given healthcare dataset, and processing lag may result from limitations on technological resources such as availability of processing, memory, storage, or bandwidth resources.

The lookback window comprises the time period for which the medical histories of the patients are observed in the ETL pipeline 305. Illustratively, and without limitations of the scope of the invention, parameters for the ETL pipeline include 12 months for the lookback window, three months for the prediction window, and one month for the offset window. The offset between RCS #1 and RCS #2 is illustratively three months (i.e., the lookback window for RCS #1 begins in October 2016 and the lookback window for RCS #2 begins three months later in January 2017). Different parameters may be utilized to meet the needs of other implementations of the present unbiased ETL system. For example, RCS #2 is rolled ahead by three months in the ETL pipeline in this illustrative embodiment, but it may be desirable to shorten or lengthen the rolling period to suit a particular deployment scenario.

Star symbols (representatively indicated by reference numeral 335) provide positive event of interest indicators for patients A, B, and C. Depending on the application, the positive event of interest can either be defined as an event that can only occur once in a patient's medical history (e.g., the first occurrence of the diagnosis for an autoimmune disease, or transition to a new medication or therapy), or an event that can reoccur (e.g., heart attack). The diagram in FIG 3 assumes the latter application and patient A has two occurrences of the event of interest. In any RCS, the patient is considered positive if the positive event of interest is occurring in the prediction window for that RCS.

As shown in FIG 3, in RCS #1, patients A and C have their positive event of interest in the prediction window and are positive for that RCS. Patients B and D are negative. In RCS #2, patient B has a positive event of interest in the prediction window and is positive for this RCS. All other patients are negative. While the medical histories for each patient in the ETL pipeline 305 are the same in each section 310 and 315, as the prediction window 320 is different for each RCS, the data used to drive the prediction model changes. After the cohorts for each RCS are created for the entire study period, positive and negative events from all of the RCS instances may be used to form the master cohort used for the prediction modeling in a particular deployment.

An illustrative implementation of RCS may utilize a software package developed in Python and PySpark. This package queries a Hadoop distributed file system (HDFS) of patient-level timestamp records for diagnosis, procedure, and prescription medical claims. PySpark may be utilized to extract relevant de-identified patient IDs and to then build a patient-level RCS table. The following illustrative, non-limiting inputs may be utilized in an exemplary iteration:
a) Inclusion criteria comprised of qualifying diagnosis and/or treatment claims, e.g., myocardial infarction
b) The study window, e.g., from November 2016 up to January 2018
c) The number of desired cross-sections, e.g., 12
d) The duration of each cross-section lookback window, e.g., 12 months
e) The duration of each cross-section offset window, e.g., one month
f) The duration of each cross-section prediction window, e.g., one month
g) The time between cross-sections, e.g., one month
h) The end date of the most recent cross-section, e.g., January 2018

The software first queries the patient claims data to generate an HDFS table of de-identified patient IDs using inputs (a) and (b), herein referred to as the initial data pull (IDP). The IDP includes all patients that satisfy our inclusion criteria (a) during the study window (b). The software then uses patient IDs from the IDP and information from (b)-(h) to generate an HDFS RCS table. Each row in the RCS table contains a single patient ID and timestamp columns that define a single RCS (i.e., columns: lookback window start and end dates, offset window start and end dates, and outcome window start and end dates).

The RCS table contains all patient cross-sections that satisfy inputs (b)-(h) and is rolling such that for a single patient the most recent cross-section is anchored in relation to (h) and the timestamps for each subsequent cross-section are shifted by (g). To label each patient cross-section with an outcome label of interest, for example, positive or negative in the case of binary classification, logic may then be applied using PySpark to query the patient claims data to evaluate whether the positive indicator (e.g., a diagnosis or treatment of interest) occurs during each cross-section prediction window. This methodology is extensible such that additional filtering criteria may be applied to refine an RCS cohort. For example, the filtering may be applied to only patient cross-sections that satisfy inclusion criteria during the lookback window and/or drop patient cross-sections where the positive indicator occurs during the lookback window.

As discussed above, the prediction window defines a period of time over which the machine learning model is looking for an event of interest such as disease progression or change of therapy. Machine learning models may be trained with a relatively narrow window such that predictions of an event of interest are imminent. Such approach can be reasonable as patient history close to the event is often highly predictable. For example, a medical procedure may commonly be performed before initiation of a new medication to assess suitability. A narrow window, however, may not always represent an optimal time period over which the machine learning model can reasonably predict patient transition, as the narrow window constrains the model to a highly select point in the patient's medical experience.

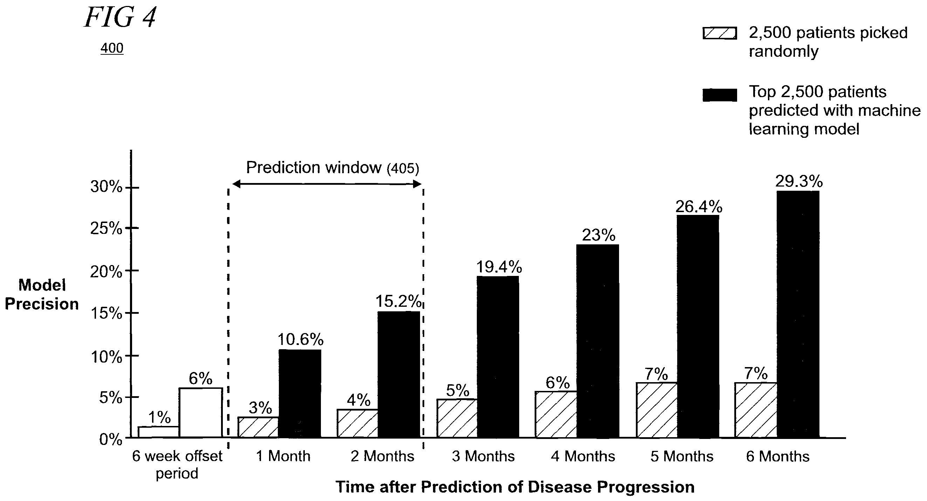

FIG 4 is a graph 400 that shows how model precision is increased as the prediction window 405 is lengthened for an illustrative real-world example of disease progression in patients with an autoimmune condition. In this example, the machine learning model was trained to predict a therapy transition within a narrow outcome window of two months. However, there may not be sufficient information in these patients' medical histories to make such a narrowly timed prediction. The precision of the model is relatively low, as shown, within a narrow prediction outcome window at 15.2% precision for the top 2,500 patients predicted by the model.

Assessing performance in this way can underestimate the usefulness of the machine learning model because a substantial percentage of false positive patients within the constrained time window do, in fact, transition to a therapy of interest when the outcome window is relaxed to six months beyond the time of prediction. As shown, precision increases to 29.3% for the same set of 2,500 patients. The precision value is more meaningful when compared to a baseline of performance in the absence of the machine learning model. In the case of the machine learning model presented here, model precision was four to five times better than selecting patients at random for disease progression which is a substantial increase over baseline.

Machine learning model precision may be measured at the patient level as discussed above, where the performance is quantified by how many patients the model identifies correctly through its predictions. However, in some commercial targeting scenarios, it is not only an effective prediction of patient events that may be important, but also of prediction events that are related to health care providers (HCP). Accordingly, predicting whether an HCP will transition a patient to a new therapy or medication may also be an important factor in validating a model's performance and applicability in a real-world and/or commercial setting.

In a similar way as with the machine learning model developed for disease progression predictions in the autoimmune example discussed above in the text accompanying FIG 4, the measurement of HCP-level precision may be constructed by defining the number of predicted patients that are linked to an HCP and rebuilding the precision measurement across expanded outcome windows. Constructed this way, an HCP-level performance assessment demonstrates that the machine learning model can achieve high levels of precision.

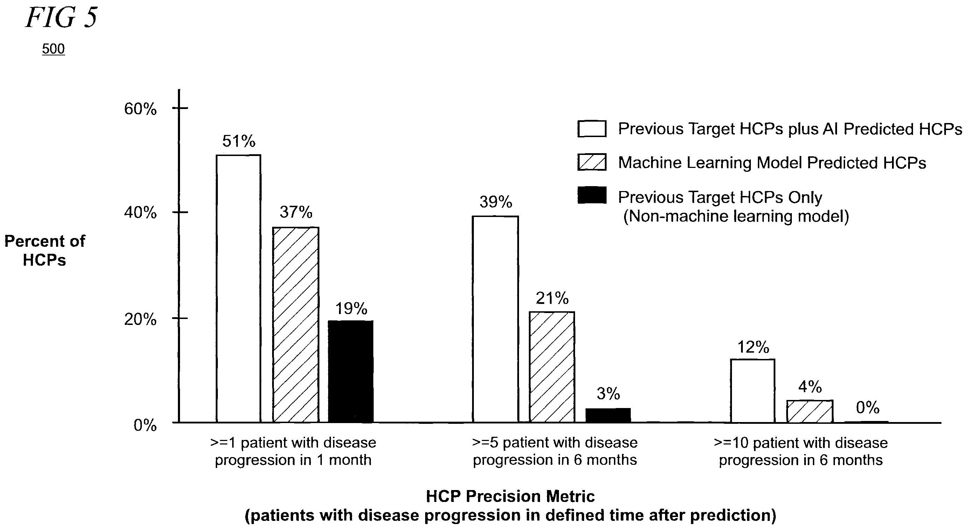

FIG 5 is a graph 500 that illustratively shows model precision in predicting when HCPs will transition a patient to a specific new therapy within the model's prediction window. This view of precision at the HCP-level defines the proportion of potential target HCPs that are expected to actually transition a predicted patient. For commercial applications, the set of HCPs identified by the machine learning model could be targeted with 37% precision, meaning that two out of five targeted HCPs would be expected to act on the disease progression and transition a predicted patient in a real-world setting within one month of the prediction, as shown.

In some real-world deployments of the present unbiased ETL system, the machine learning model may be subject to ongoing re-optimization after being trained on specific historical data. Through this process, a predictive model can be provided with more recent data, including additional positive patients, or those patients that have the outcome of interest, as well as updated timing and market influences or changes.

Two main options can be implemented for ongoing re-optimization of a model, including: 1) refreshing with newer data, and 2) refreshing with data collected prospectively. For the first option, on a routine basis, the model can be updated with additional data that is collected between the initial predictions and the new round of predictions. For the second option, an additional model can be developed to track previously predicted positives to understand how many of the predicted patients ultimately experience disease progression.

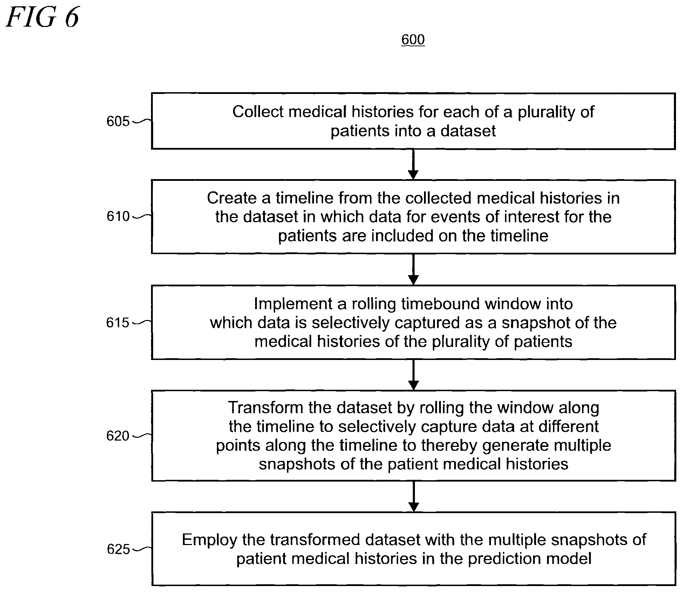

FIG 6 is a flowchart of an illustrative method 600 that may be performed by a computing device such as a personal computer, workstation, or server to implement the present unbiased ETL system for timed medical event prediction. Unless specifically stated, the methods or steps shown in the flowcharts and described in the accompanying text are not constrained to a particular order or sequence. In addition, some of the methods or steps thereof can occur or be performed concurrently and not all the methods or steps have to be performed in a given implementation depending on the requirements of such implementation and some methods or steps may be optionally utilized.

In step 605, medical histories are collected for each of a plurality of patients into a dataset. In step 610, a timeline is created from the collected medical histories in the dataset in which data for events of interest for the patients are included on the timeline. In step 615, a rolling timebound window is implemented into which data is selectively captured as a snapshot of the medical histories of the plurality of patients. In step 620, the dataset is transformed by rolling the window along the timeline to selectively capture data at different points along the timeline to thereby generate multiple snapshots of the patient medical histories. In step 625, the transformed dataset with the multiple snapshots of patient medical histories is employed in the prediction model.

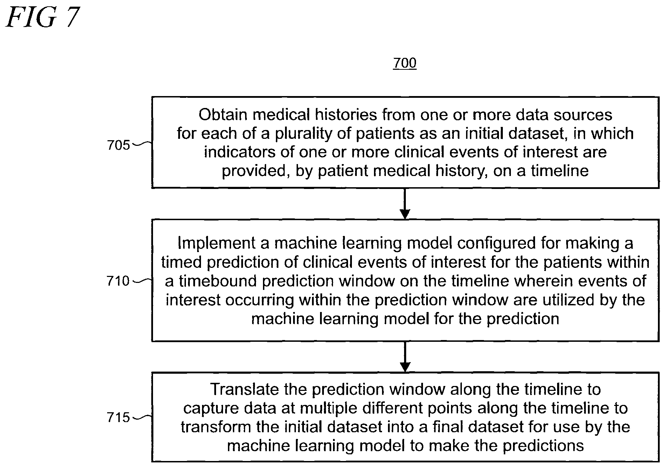

FIG 7 is a flowchart of an illustrative method 700 that may be performed by a computing device such as a personal computer, workstation, or server to implement the present unbiased ETL system for timed medical event prediction. In step 705, medical histories are obtained from one or more data sources for each of a plurality of patients as an initial dataset, in which indicators of one or more clinical events of interest are provided, by patient medical history, on a timeline. In step 710, a machine learning model is implemented that is configured for making a timed prediction of events of interest for patients within a timebound prediction window on the timeline wherein clinical events of interest occurring within the prediction window are utilized by the machine learning model for the event prediction. In step 715, the prediction window is translated along the timeline to capture data at multiple different points along the timeline to transform the initial dataset into a final dataset for use by the machine learning model to make the predictions.

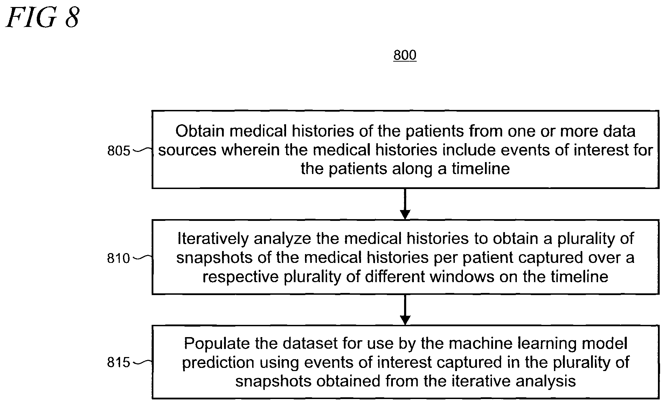

FIG 8 is a flowchart of an illustrative method 800 that may be performed by a computing device such as a personal computer, workstation, or server to implement the present unbiased ETL system for timed medical event prediction. In step 805, medical histories of the patients are obtained from one or more data sources wherein the medical histories include events of interest for the patients along a timeline. In step 810, the medical histories are iteratively analyzed to obtain a plurality of snapshots of the medical histories per patient captured over a respective plurality of different windows on the timeline. In step 815, the dataset is populated for use by the machine learning prediction model using events of interest captured in the plurality of snapshots obtained from the iterative analysis.

FIG 9 is a simplified block diagram of an illustrative architecture of a computer system 900 such as a PC or server with which the present unbiased ETL system for timed medical event prediction may be implemented. Computer system 900 includes a processor 905, a system memory 911, and a system bus 914 that couples various system components including the system memory 911 to the processor 905. The system bus 914 may be any of several types of bus structures including a memory bus or memory controller, a peripheral bus, or a local bus using any of a variety of bus architectures. The system memory 911 includes read only memory (ROM) 917 and random-access memory (RAM) 921. A basic input/output system (BIOS) 925, containing the basic routines that help to transfer information between elements within the computer system 900, such as during startup, is stored in ROM 917. The computer system 900 may further include a hard disk drive 928 for reading from and writing to an internally disposed hard disk (not shown), a magnetic disk drive 930 for reading from or writing to a removable magnetic disk 933 (e.g., a floppy disk), and an optical disk drive 938 for reading from or writing to a removable optical disk 943 such as a CD (compact disc), DVD (digital versatile disc), or other optical media. The hard disk drive 928, magnetic disk drive 930, and optical disk drive 938 are connected to the system bus 914 by a hard disk drive interface 946, a magnetic disk drive interface 949, and an optical drive interface 952, respectively. The drives and their associated computer-readable storage media provide non-volatile storage of computer-readable instructions, data structures, program modules, and other data for the computer system 900. Although this illustrative example includes a hard disk, a removable magnetic disk 933, and a removable optical disk 943, other types of computer-readable storage media which can store data that is accessible by a computer such as magnetic cassettes, Flash memory cards, digital video disks, data cartridges, random access memories (RAMs), read only memories (ROMs), and the like may also be used in some applications of the present unbiased ETL system for timed medical event prediction. In addition, as used herein, the term computer-readable storage media includes one or more instances of a media type (e.g., one or more magnetic disks, one or more CDs, etc.). For purposes of this specification and the claims, the phrase "computer-readable storage media" and variations thereof, are intended to cover non-transitory embodiments, and do not include waves, signals, and/or other transitory and/or intangible communication media.

A number of program modules may be stored on the hard disk, magnetic disk 933, optical disk 943, ROM 917, or RAM 921, including an operating system 955, one or more application programs 957, other program modules 960, and program data 963. A user may enter commands and information into the computer system 900 through input devices such as a keyboard 966 and pointing device 968 such as a mouse. Other input devices (not shown) may include a microphone, joystick, game pad, satellite dish, scanner, trackball, touchpad, touchscreen, touch-sensitive device, voice-command module or device, user motion or user gesture capture device, or the like. These and other input devices are often connected to the processor 905 through a serial port interface 971 that is coupled to the system bus 914, but may be connected by other interfaces, such as a parallel port, game port, or universal serial bus (USB). A monitor 973 or other type of display device is also connected to the system bus 914 via an interface, such as a video adapter 975. In addition to the monitor 973, personal computers typically include other peripheral output devices (not shown), such as speakers and printers. The illustrative example shown in FIG 9 also includes a host adapter 978, a Small Computer System Interface (SCSI) bus 983, and an external storage device 976 connected to the SCSI bus 983.

The computer system 900 is operable in a networked environment using logical connections to one or more remote computers, such as a remote computer 988. The remote computer 988 may be selected as another personal computer, a server, a router, a network PC, a peer device, or other common network node, and typically includes many or all of the elements described above relative to the computer system 900, although only a single representative remote memory/storage device 990 is shown in FIG 9. The logical connections depicted in FIG 9 include a local area network (LAN) 993 and a wide area network (WAN) 995. Such networking environments are often deployed, for example, in offices, enterprise-wide computer networks, intranets, and the Internet.

When used in a LAN networking environment, the computer system 900 is connected to the local area network 993 through a network interface or adapter 996. When used in a WAN networking environment, the computer system 900 typically includes a broadband modem 998, network gateway, or other means for establishing communications over the wide area network 995, such as the Internet. The broadband modem 998, which may be internal or external, is connected to the system bus 914 via a serial port interface 971. In a networked environment, program modules related to the computer system 900, or portions thereof, may be stored in the remote memory/storage device 990. It is noted that the network connections shown in FIG 9 are illustrative and other means of establishing a communications link between the computers may be used depending on the specific requirements of an application of the unbiased ETL system for timed medical event prediction.

FIG 10 shows an illustrative architecture 1000 for a client computing device such as a laptop computer or personal computer for the present unbiased ETL system for timed medical event prediction. The architecture 1000 illustrated in FIG 10 includes one or more processors 1002 (e.g., central processing unit, dedicated Artificial Intelligence chip, graphics processing unit, etc.), a system memory 1004, including RAM (random access memory) 1006 and ROM (read only memory) 1008, and a system bus 1010 that operatively and functionally couples the components in the architecture 1000. A basic input/output system containing the basic routines that help to transfer information between elements within the architecture 1000, such as during startup, is typically stored in the ROM 1008. The architecture 1000 further includes a mass storage device 1012 for storing software code or other computer-executed code that is utilized to implement applications, the file system, and the operating system. The mass storage device 1012 is connected to the processor 1002 through a mass storage controller (not shown) connected to the bus 1010. The mass storage device 1012 and its associated computer-readable storage media provide non-volatile storage for the architecture 1000. Although the description of computer-readable storage media contained herein refers to a mass storage device, such as a hard disk or CD-ROM drive, it may be appreciated by those skilled in the art that computer-readable storage media can be any available storage media that can be accessed by the architecture 1000.

By way of example, and not limitation, computer-readable storage media may include volatile and non-volatile, removable and non-removable media implemented in any method or technology for storage of information such as computer-readable instructions, data structures, program modules, or other data. For example, computer-readable media includes, but is not limited to, RAM, ROM, EPROM (erasable programmable read only memory), EEPROM (electrically erasable programmable read only memory), Flash memory or other solid state memory technology, CD-ROM, DVD, HD-DVD (High Definition DVD), Blu-ray or other optical storage, magnetic cassette, magnetic tape, magnetic disk storage or other magnetic storage device, or any other medium which can be used to store the desired information and which can be accessed by the architecture 1000.

According to various embodiments, the architecture 1000 may operate in a networked environment using logical connections to remote computers through a network. The architecture 1000 may connect to the network through a network interface unit 1016 connected to the bus 1010. It may be appreciated that the network interface unit 1016 also may be utilized to connect to other types of networks and remote computer systems. The architecture 1000 also may include an input/output controller 1018 for receiving and processing input from a number of other devices, including a keyboard, mouse, touchpad, touchscreen, control devices such as buttons and switches or electronic stylus (not shown in FIG 10). Similarly, the input/output controller 1018 may provide output to a display screen, user interface, a printer, or other type of output device (also not shown in FIG 10).

It may be appreciated that the software components described herein may, when loaded into the processor 1002 and executed, transform the processor 1002 and the overall architecture 1000 from a general-purpose computing system into a special-purpose computing system customized to facilitate the functionality presented herein. The processor 1002 may be constructed from any number of transistors or other discrete circuit elements, which may individually or collectively assume any number of states. More specifically, the processor 1002 may operate as a finite-state machine, in response to executable instructions contained within the software modules disclosed herein. These computer-executable instructions may transform the processor 1002 by specifying how the processor 1002 transitions between states, thereby transforming the transistors or other discrete hardware elements constituting the processor 1002.

Encoding the software modules presented herein also may transform the physical structure of the computer-readable storage media presented herein. The specific transformation of physical structure may depend on various factors in different implementations of this description. Examples of such factors may include, but are not limited to, the technology used to implement the computer-readable storage media, whether the computer-readable storage media is characterized as primary or secondary storage, and the like. For example, if the computer-readable storage media is implemented as semiconductor-based memory, the software disclosed herein may be encoded on the computer-readable storage media by transforming the physical state of the semiconductor memory. For example, the software may transform the state of transistors, capacitors, or other discrete circuit elements constituting the semiconductor memory. The software also may transform the physical state of such components in order to store data thereupon.

As another example, the computer-readable storage media disclosed herein may be implemented using magnetic or optical technology. In such implementations, the software presented herein may transform the physical state of magnetic or optical media, when the software is encoded therein. These transformations may include altering the magnetic characteristics of particular locations within given magnetic media. These transformations also may include altering the physical features or characteristics of particular locations within given optical media to change the optical characteristics of those locations.

In light of the above, it may be appreciated that many types of physical transformations take place in the architecture 1000 in order to store and execute the software components presented herein. It also may be appreciated that the architecture 1000 may include other types of computing devices, including wearable devices, handheld computers, embedded computer systems, smartphones, PDAs, and other types of computing devices known to those skilled in the art. It is also contemplated that the architecture 1000 may not include all of the components shown in FIG 10, may include other components that are not explicitly shown in FIG 10, or may utilize an architecture completely different from that shown in FIG 10.

## Claims

1. A computing device (900) configured to support an ETL system (100) supporting a timed medical prediction model (110) using machine learning, comprising:
one or more processors (905); and
one or more hardware-based non-transitory computer-readable memory devices (928, 933, 943) storing instructions which, when executed by the one or more processors (905), cause the computing device (900) to conduct the steps:
collect (605) medical histories for each of a plurality of patients into a dataset;
create (610) a timeline from the collected medical histories in the dataset in which data for events of interest for the patients are included on the timeline, wherein the events of interest comprise at least one of diagnosis of a disease, progression of a disease, initiation of treatment or therapy, discontinuation of treatment or therapy, occurrence of an adverse event, favorable response to treatment, hospitalization, readmission;
implement (615) a rolling timebound window (215, 220, 230) into which data is selectively captured as a snapshot of the medical histories of the plurality of patients;
transform (620) the dataset by rolling the window (215, 220, 230) along the timeline to selectively capture data at different points along the timeline to thereby generate multiple snapshots (205; 310, 315) of the patient medical histories, wherein the multiple snapshots (205; 310, 315) are captured to show a change in therapeutic status from an earlier time to a later time, and
employ (625) the transformed dataset with the multiple snapshots (205; 310, 315) of patient medical histories in the prediction model (110) to predict one or more medical events of interest,
wherein the rolling window (215, 220, 230) comprises a lookback window (215; 330) and a prediction window (220; 320), wherein the prediction window (220; 320) defines the time period over which an event of interest is predicted, wherein the lookback window (215; 330) precedes the prediction window (220; 320) on the timeline and the events of interest occurring within the lookback window (215; 330) are utilized for training of the prediction model (110), wherein the generated multiple snapshots (205; 310, 315) are utilized to increase sample size.

2. The computing device (900) of claim 1 in which the events of interest indicate disease progression or change of therapy and the dataset of selectively captured data comprises positive events of interest.

3. The computing device (900) of claim 1 in which the multiple snapshots (205; 310, 315) of patient medical histories enable analysis of a given patient at multiple different points along the timeline.

4. The computing device (900) of claim 1 further comprising labelling patients as belonging to one of multiple classes depending on current healthcare status.

5. The computing device (900) of claim 4 further comprising storing the predictions in a destination system (120) that includes a user interface (140) configured to enable users (125) to interact with the stored predictions (115).

6. The computing device (900) of claim 1 in which the rolling window (215, 220, 230) further comprises an offset window (230) comprising a time period prior to the prediction window (220) and incorporated to accommodate lags in data collection (605).

7. One or more hardware-based non-transitory computer-readable memory devices (928, 933, 943) storing instructions which, when executed by one or more processors (905) disposed in a computing device (900), cause the computing device (900) to conduct the steps as defined in claim 1.

8. The one or more hardware-based non-transitory computer-readable memory devices (928, 933, 943) of claim 7 in which the final dataset (310, 315) having data captured at multiple different points along the timeline has reduced sampling bias relative to the initial dataset (305), wherein the sampling bias results from one of seasonality, changes in data coverage, or changes in market conditions.

9. The one or more hardware-based non-transitory computer-readable memory devices (928, 933, 943) of claim 7 in which the cross-section window further comprises an offset window (325) immediately preceding the prediction window (220; 320) on the timeline, the cross-section window comprising a section of the timeline having a predetermined length, in which the offset window (325) is chosen to accommodate time lags in data collection from the data sources (105).

10. The one or more hardware-based non-transitory computer-readable memory devices (928, 933, 943) of claim 7 in which the executed instructions further cause the computing device (900) to utilize the final dataset (310, 315) to validate the machine learning model (110) or test the machine learning model (110).

11. The one or more hardware-based non-transitory computer-readable memory devices (928, 933, 943) of claim 7 in which the executed instructions further cause the computing device (900) to use different portions of the final dataset (310, 315) for training to thereby compensate for drift of the machine learning model (110) or bias in the machine learning model (110).

12. A method (800) implemented on a computing device (900) for generating a dataset for utilization in a machine learning model (110) that is configured to predict one or more medical events of interest for medical patients, the method (800) comprising the steps as defined in claim 1.

13. The method (800) of claim 12 in which the different windows have offset start times and end times on the timeline and the events of interest relate to one of disease progression or change of therapy.

14. The method (800) of claim 12 in which each of the different windows further comprises an offset window (230; 325) between the lookback window (215; 330) and the prediction window (220; 320) on the timeline, and in which the offset window (230; 325) provides a predetermined time lag to the prediction window (220; 320) such that the machine learning model (110) is enabled to predict an event of interest by an amount of time equal to the size of the offset window (230; 325).

15. The method (800) of claim 12 further comprising storing predictions from operations of the machine learning model (110) in a destination system (120) that is configured to interface with one or more computing device users (125) to enable review and analysis of the predictions.

## Patentansprüche

1. Recheneinrichtung (900), die zur Unterstützung eines ETL-Systems (100) konfiguriert ist, welches ein zeitbasiertes medizinisches Vorhersagemodell (110) unter Verwendung von maschinellem Lernen unterstützt, umfassend:
einen oder mehrere Prozessoren (905); und
eine oder mehrere hardwarebasierte, nichtflüchtige, computerlesbare Speichereinrichtungen (928, 933, 943), die Anweisungen speichern, welche, wenn sie von dem einen oder den mehreren Prozessoren (905) ausgeführt werden, bewirken, dass die Recheneinrichtung (900) die folgenden Schritte durchführt:
Sammeln (605) von medizinischen Vorgeschichten für jeden einer Mehrzahl von Patienten in einem Datensatz;
Erstellen (610) einer Zeitachse aus den gesammelten medizinischen Vorgeschichten in dem Datensatz, in der Daten zu relevanten Ereignissen für die Patienten in der Zeitachse enthalten sind, wobei die relevanten Ereignisse mindestens eines von Folgendem umfassen: Diagnose einer Krankheit, Verlauf einer Krankheit, Beginn einer Behandlung oder Therapie, Beendigung einer Behandlung oder Therapie, Auftreten eines nachteiligen Ereignisses, günstiges Ansprechen auf eine Behandlung, Hospitalisierung, erneute Einweisung;
Implementieren (615) eines gleitenden, zeitlich begrenzten Fensters (215, 220, 230), in das Daten selektiv als Momentaufnahme der medizinischen Vorgeschichten der mehreren Patienten erfasst werden;
Transformieren (620) des Datensatzes durch Verschieben des Fensters (215, 220, 230) entlang der Zeitachse, um selektiv Daten an verschiedenen Punkten entlang der Zeitachse zu erfassen und dadurch mehrere Momentaufnahmen (205; 310, 315) der medizinischen Vorgeschichten der Patienten zu erzeugen, wobei die mehreren Momentaufnahmen (205; 310, 315) erfasst werden, um eine Veränderung des therapeutischen Status von einem früheren Zeitpunkt zu einem späteren Zeitpunkt darzustellen, und
Verwenden (625) des transformierten Datensatzes mit den mehreren Momentaufnahmen (205; 310, 315) der medizinischen Vorgeschichten der Patienten im Vorhersagemodell (110), um ein oder mehrere medizinische Ereignisse von Interesse vorherzusagen,
wobei das gleitende Fenster (215, 220, 230) ein Rückschaufenster (215; 330) und ein Vorhersagefenster (220; 320) umfasst, wobei das Vorhersagefenster (220; 320) den Zeitraum definiert, über den ein Ereignis von Interesse vorhergesagt wird, wobei das Rückschaufenster (215; 330) auf der Zeitachse dem Vorhersagefenster (220; 320) vorausgeht und die innerhalb des Rückschaufensters (215; 330) auftretenden Ereignisse von Interesse für das Training des Vorhersagemodells (110) verwendet werden, wobei die erzeugten mehreren Momentaufnahmen (205; 310, 315) genutzt werden, um die Stichprobengröße zu erhöhen.

2. Recheneinrichtung (900) nach Anspruch 1, wobei die relevanten Ereignisse einen Krankheitsverlauf oder eine Therapieänderung anzeigen und der Datensatz aus selektiv erfassten Daten positive relevante Ereignisse umfasst.

3. Recheneinrichtung (900) nach Anspruch 1, wobei die mehreren Momentaufnahmen (205; 310, 315) der medizinischen Vorgeschichten der Patienten eine Analyse eines bestimmten Patienten an mehreren verschiedenen Zeitpunkten entlang der Zeitachse ermöglichen.

4. Recheneinrichtung (900) nach Anspruch 1, ferner umfassend eine Kennzeichnung von Patienten als zu einer von mehreren Klassen zugehörig, abhängig vom aktuellen Gesundheitszustand.

5. Recheneinrichtung (900) nach Anspruch 4, ferner umfassend ein Speichern der Vorhersagen in einem Zielsystem (120), das eine Benutzerschnittstelle (140) umfasst, die dazu konfiguriert ist, Benutzern (125) eine Interaktion mit den gespeicherten Vorhersagen (115) zu ermöglichen.

6. Recheneinrichtung (900) nach Anspruch 1, wobei das gleitende Fenster (215, 220, 230) ferner ein Offset-Fenster (230) umfasst, das einen Zeitraum vor dem Vorhersagefenster (220) umfasst und vorgesehen ist, um Verzögerungen bei der Datenerfassung (605) auszugleichen.

7. Eine oder mehrere hardwarebasierte, nichtflüchtige, computerlesbare Speichereinrichtungen (928, 933, 943), die Anweisungen speichern, welche, wenn sie von einem oder mehreren in einer Recheneinrichtung (900) angeordneten Prozessoren (905) ausgeführt werden, bewirken, dass die Recheneinrichtung (900) die in Anspruch 1 definierten Schritte durchführt.

8. Eine oder mehrere hardwarebasierten, nichtflüchtige, computerlesbare Speichereinrichtungen (928, 933, 943) nach Anspruch 7, wobei der endgültige Datensatz (310, 315) mit Daten, die an mehreren verschiedenen Zeitpunkten entlang der Zeitachse erfasst wurden, im Vergleich zum ursprünglichen Datensatz (305) eine verringerte Stichprobenverzerrung aufweist, wobei die Stichprobenverzerrung auf saisonale Schwankungen, Änderungen der Datenabdeckung oder Änderungen der Marktbedingungen zurückzuführen ist.

9. Eine oder mehrere hardwarebasierte, nichtflüchtige, computerlesbare Speichereinrichtungen (928, 933, 943) nach Anspruch 7, wobei das Querschnittsfenster ferner ein Offset-Fenster (325) umfasst, das dem Vorhersagefenster (220; 320) auf der Zeitachse unmittelbar vorangeht, wobei das Querschnittsfenster einen Abschnitt der Zeitachse mit einer vorbestimmten Länge umfasst, wobei das Offset-Fenster (325) so gewählt ist, dass es Zeitverzögerungen bei der Datenerfassung aus den Datenquellen (105) ausgleicht.

10. Eine oder mehrere hardwarebasierte, nichtflüchtige, computerlesbare Speichereinrichtungen (928, 933, 943) nach Anspruch 7, wobei die ausgeführten Anweisungen ferner bewirken, dass die Recheneinrichtung (900) den endgültigen Datensatz (310, 315) verwendet, um das Maschinenlernmodell (110) zu validieren oder das Maschinenlernmodell (110) zu testen.

11. Eine oder mehrere hardwarebasierte, nichtflüchtige, computerlesbare Speichereinrichtungen (928, 933, 943) nach Anspruch 7, wobei die ausgeführten Anweisungen ferner bewirken, dass die Recheneinrichtung (900) verschiedene Teile des endgültigen Datensatzes (310, 315) für das Training verwendet, um dadurch eine Drift des Maschinenlernmodells (110) oder eine Verzerrung im Maschinenlernmodell (110) auszugleichen.

12. Verfahren (800), das auf einer Recheneinrichtung (900) implementiert ist, zum Erzeugen eines Datensatzes zur Verwendung in einem Maschinenlernmodell (110), das dazu konfiguriert ist, ein oder mehrere medizinische Ereignisse von Interesse für medizinische Patienten vorherzusagen, wobei das Verfahren (800) die in Anspruch 1 definierten Schritte umfasst.

13. Verfahren (800) nach Anspruch 12, wobei die verschiedenen Fenster versetzte Start- und Endzeitpunkte auf der Zeitachse aufweisen und sich die Ereignisse von Interesse einen Krankheitverlauf oder eine Therapieänderung betreffen.

14. Verfahren (800) nach Anspruch 12, wobei jedes der verschiedenen Fenster ferner ein Offset-Fenster (230; 325) zwischen dem Rückschaufenster (215; 330) und dem Vorhersagefenster (220; 320) auf der Zeitachse umfasst und wobei das Offset-Fenster (230; 325) eine vorbestimmte Zeitverzögerung gegenüber dem Vorhersagefenster (220; 320) vorsieht, so dass dem Maschinenlernmodell (110) ermöglicht wird, ein Ereignis von Interesse um einen Zeitraum vorherzusagen, der der Größe des Offset-Fensters (230; 325) entspricht.

15. Verfahren (800) nach Anspruch 12, ferner umfassend einSpeichern von Vorhersagen aus Operationen des Maschinenlernmodells (110) in einem Zielsystem (120), das dazu konfiguriert ist, mit einem oder mehreren Recheneinrichtungsbenutzern (125) zu kommunizieren, um eine Überprüfung und Analyse der Vorhersagen zu ermöglichen.

## Revendications

1. Dispositif informatique (900) configuré pour supporter un système ETL (100) supportant un modèle de prédiction médicale temporelle (110) utilisant l'apprentissage automatique, comprenant :
un ou plusieurs processeurs (905) ; et
un ou plusieurs dispositifs de mémoire non volatils, basés sur le matériel, lisibles par ordinateur (928, 933, 943) stockant des instructions qui, lorsqu'elles sont exécutées par le ou les processeurs (905), amènent le dispositif informatique (900) à effectuer les étapes suivantes :
collecter (605) des antécédents médicaux pour chacun d'une pluralité de patients dans un ensemble de données ;
créer (610) une ligne temporelle à partir des antécédents médicaux collectés dans l'ensemble de données, dans laquelle sont incluses les données des événements d'intérêt pour les patients sur la ligne temporelle, les événements d'intérêt comprenant au moins l'un des éléments suivants : diagnostic d'une maladie, progression d'une maladie, début d'un traitement ou d'une thérapie, arrêt d'un traitement ou d'une thérapie, survenue d'un événement indésirable, réponse favorable au traitement, hospitalisation, réadmission ;
mettre en œuvre (615) une fenêtre glissante limitée dans le temps (215, 220, 230) dans laquelle les données sont capturées de manière sélective sous la forme d'un instantané des antécédents médicaux de la pluralité de patients ;
transformer (620) l'ensemble de données en faisant glisser la fenêtre (215, 220, 230) le long de la ligne temporelle afin de capturer de manière sélective des données à différents points de la ligne temporelle, pour ainsi générer plusieurs instantanés (205 ; 310, 315) des antécédents médicaux des patients, où lesdits plusieurs instantanés (205 ; 310, 315) sont capturés pour indiquer un changement de l'état thérapeutique entre un moment antérieur et un moment postérieur, et
utiliser (625) l'ensemble de données transformé comprenant les différents instantanés (205 ; 310, 315) des antécédents médicaux des patients dans le modèle de prédiction (110) afin de prédire un ou plusieurs événements médicaux d'intérêt,
dans lequel la fenêtre glissante (215, 220, 230) comprend une fenêtre rétrospective (215 ; 330) et une fenêtre de prédiction (220 ; 320), la fenêtre de prédiction (220 ; 320) définit la période de temps sur laquelle un événement d'intérêt est prédit, la fenêtre rétrospective (215 ; 330) précédant la fenêtre de prédiction (220 ; 320) sur la ligne temporelle et les événements d'intérêt se produisant dans la fenêtre rétrospective (215 ; 330) étant utilisés pour l'apprentissage du modèle de prédiction (110), les multiples instantanés générés (205 ; 310, 315) sont utilisés pour augmenter la taille de l'échantillon.

2. Dispositif informatique (900) selon la revendication 1, dans lequel les événements d'intérêt indiquent la progression d'une maladie ou un changement de traitement, et l'ensemble de données capturées de manière sélective comprend des événements d'intérêt positifs.

3. Dispositif informatique (900) selon la revendication 1, dans lequel les multiples instantanés (205 ; 310, 315) des antécédents médicaux des patients permettent d'analyser un patient donné à plusieurs moments différents le long de la ligne temporelle.

4. Dispositif informatique (900) selon la revendication 1, comprenant en outre un classement des patients dans l'une de plusieurs classes en fonction de leur état de santé actuel.

5. Dispositif informatique (900) selon la revendication 4, comprenant en outre un stockage des prédictions dans un système de destination (120) qui comprend une interface utilisateur (140) configurée pour permettre aux utilisateurs (125) d'interagir avec les prédictions stockées (115).

6. Dispositif informatique (900) selon la revendication 1, dans lequel la fenêtre glissante (215, 220, 230) comprend en outre une fenêtre de décalage (230) couvrant une période antérieure à la fenêtre de prédiction (220) et intégrée pour tenir compte des retards dans la collecte des données (605).

7. Un ou plusieurs dispositifs de mémoire non volatils, basés sur le matériel, lisibles par ordinateur (928, 933, 943) stockant des instructions qui, lorsqu'elles sont exécutées par un ou plusieurs processeurs (905) disposés dans un dispositif informatique (900), amènent le dispositif informatique (900) à effectuer les étapes définies dans la revendication 1.

8. Le ou les dispositifs de mémoire non volatils, basés sur le matériel, lisibles par ordinateur (928, 933, 943) de la revendication 7, dans lesquels l'ensemble de données final (310, 315) comportant des données capturées en plusieurs points différents le long de la ligne temporelle présente un biais d'échantillonnage réduit par rapport à l'ensemble de données initial (305), le biais d'échantillonnage résultant soit d'une saisonnalité, soit de changements dans la couverture des données, ou soit de changements dans les conditions de marché.

9. Le ou les dispositifs de mémoire non volatils, basés sur le matériel, lisibles par ordinateur (928, 933, 943) selon la revendication 7, dans lesquels la fenêtre de section transversale comprend en outre une fenêtre de décalage (325) précédant immédiatement la fenêtre de prédiction (220 ; 320) sur la ligne temporelle, la fenêtre de section transversale comprenant une section de la ligne temporelle ayant une longueur prédéterminée, la fenêtre de décalage (325) étant choisie pour tenir compte des décalages temporels dans la collecte des données provenant des sources de données (105).

10. Le ou les dispositifs de mémoire non volatils, basés sur le matériel, lisibles par ordinateur (928, 933, 943) selon la revendication 7, dans lesquels les instructions exécutées amènent en outre le dispositif informatique (900) à utiliser l'ensemble de données final (310, 315) pour valider le modèle d'apprentissage automatique (110) ou tester le modèle d'apprentissage automatique (110).

11. Le ou les dispositifs de mémoire non volatils, basés sur le matériel, lisibles par ordinateur (928, 933, 943) selon la revendication 7, dans lesquels les instructions exécutées amènent en outre le dispositif informatique (900) à utiliser différentes parties de l'ensemble de données final (310, 315) pour l'entraînement, afin de compenser ainsi la dérive du modèle d'apprentissage automatique (110) ou le biais dans le modèle d'apprentissage automatique (110).

12. Procédé (800) mis en œuvre sur un dispositif informatique (900) pour générer un ensemble de données pour utilisation dans un modèle d'apprentissage automatique (110) qui est configuré pour prédire un ou plusieurs événements médicaux d'intérêt pour des patients, le procédé (800) comprenant les étapes définies dans la revendication 1.

13. Procédé (800) selon la revendication 12, dans lequel les différentes fenêtres présentent des temps de début et de fin décalés sur la ligne temporelle et les événements d'intérêt concernant une progression de maladie ou un changement de traitement.

14. Procédé (800) selon la revendication 12, dans lequel chacune des différentes fenêtres comprend en outre une fenêtre de décalage (230 ; 325) entre la fenêtre rétrospective (215 ; 330) et la fenêtre de prédiction (220 ; 320) sur la ligne temporelle, et dans lequel la fenêtre de décalage (230 ; 325) introduit un décalage temporel prédéterminé par rapport à la fenêtre de prédiction (220 ; 320), de sorte que le modèle d'apprentissage automatique (110) peut prédire un événement d'intérêt avec une longueur de temps égale à la taille de la fenêtre de décalage (230 ; 325).

15. Procédé (800) selon la revendication 12, comprenant en outre un stockage des prédictions issues des opérations du modèle d'apprentissage automatique (110) dans un système de destination (120) configuré pour s'interfacer avec un ou plusieurs utilisateurs de dispositif informatique (125) afin de permettre d'examiner et d'analyser les prédictions.
